# FASCICULE DE BREVET EUROPEEN

(11) **EP 2 285 391 B1**
(45) Date de publication et mention de la délivrance du brevet: **05.03.2014**
(21) Numéro de dépôt: 09746003.4
(22) Date de dépôt: 28.04.2009
(51) Int. Cl.: A61K 36/48, A23L 1/30, A23K 1/18, A23K 1/16

(54) **EXTRAIT DE FENUGREC POUR LE TRAITEMENT DES PATHOLOGIES HUMAINES ET ANIMALES IMPLIQUANT DES PARASITES FLAGELLÉS**
FENCHELEXTRAKT ZUR BEHANDLUNG VON MENSCHLICHEN UND TIERISCHEN ERKRANKUNGEN IM ZUSAMMENHANG MIT FLAGELLATA-PARASITEN
FENUGREEK EXTRACT FOR TREATING HUMAN AND ANIMAL DISEASES INVOLVING FLAGELLATE PARASITES

(30) Priorité: 13.05.2008 FR 0853068
(43) Date de publication de la demande: 23.02.2011
(73) Titulaire: Setubio Sas, 03270 Hauterive (FR)
(72) Inventeur: SERGERE, Jean-Christophe, F-03140 Etroussat (FR); VIVARES, Christian, F-63000 Clermont Ferrand (FR)
(74) Mandataire: Denjean, Eric
(86) Numéro de dépôt international: PCT/FR2009/050780
(87) Numéro de publication internationale: WO 2009/138684

(56) Documents cités:
- WO-A-2008/003007
- FR-A- 2 833 813
- DATABASE WPI Week 2008 Thomson Scientific, London, GB; AN 2008-C41862 XP002510129 "Mixed feed manufacture for reducing incidence rate of diarrhea in young age piglets" & JP 2008 011731 A (KYODO SHRYO CO LTD) 24 janvier 2008 (2008-01-24)
- FRANCIS G ET AL: "THE BIOLOGICAL ACTION OF SAPONINS IN ANIMAL SYSTEMS: A REVIEW" BRITISH JOURNAL OF NUTRITION, CAMBRIDGE UNIVERSITY PRESS, CAMBRIDGE, GB, vol. 88, no. 6, 1 décembre 2002 (2002-12-01), pages 587-605, XP008022748 ISSN: 0007-1145

## Description

L'invention a pour objet un extrait de fenugrec utilisé seul ou incorporé au sein d'un complément alimentaire ou d'une composition pharmaceutique pour le traitement des pathologies humaines ou animales dans lesquelles des parasites flagellés sont impliqués.

L'invention est plus particulièrement illustrée en relation avec les parasites flagellés, tels que *Histomonas meleagridis* et *Trichomonas vaginalis* bien qu'elle puisse s'appliquer à tous les parasites flagellés ayant une structure et une physiologie similaire à celle des parasites précités appartenant au Phylum des Metamonada.

Le parasite *Histomonas meleagridis* est responsable d'une maladie parasitaire infectieuse propre aux oiseaux galliformes appelée histomonose. Cette maladie est une typhlo-hépatite infectant particulièrement la dinde. Elle se manifeste par une diarrhée jaune soufre entraînant souvent une mortalité importante. L'observation d'une cyanose des appendices charnus de la tête fait que cette maladie porte le nom de maladie de la tête noire (black head disease). Les autres signes cliniques sont les plumes tâchées de fientes, l'anorexie, la somnolence, la démarche anormale, la tête basse ou cachée sous une aile. Une mortalité importante est observée à partir du 14^{ème} jour. En l'absence de traitement, plus de 90% des animaux peuvent mourir. Les gallinacés, en particulier les dindes qui survivent, vont présenter un retard de croissance par rapport aux dindes non atteintes cliniquement.

De nombreux traitements ont d'ores et déjà été proposés pour lutter contre l'histomonose. En particulier, les nitro-imidazoles tels que notamment le dimétridazole (DMZ) sont très actifs contre *Histomonas meleagridis.* Néanmoins, en raison de leur toxicité pour les consommateurs, cette famille de molécules a été retirée du marché. Il s'est avéré que l'albendazole et les autres benzimidazoles n'étaient pas efficaces pour traiter l'histomonose. Il en est de même avec les antibiotiques actuellement autorisés sur le marché, ainsi que les anticoccidiens tels que la roxarsone. Par ailleurs, les essais de vaccination avec des souches atténuées n'ont jamais abouti.

Compte tenu de cette situation, la seule prophylaxie envisageable consiste, soit à séparer les espèces, soit à administrer un vermifuge efficace contre le protozoaire.

En d'autres termes, le problème que se propose de résoudre l'invention réside dans la mise au point d'une composition efficace pour le traitement prophylactique et curatif, en particulier des gallinacés contre le protozoaire *Histomonas meleagridis.*

L'invention est également plus particulièrement illustrée en relation avec un protozoaire flagellé présentant une physiologie et une structure similaires à celle d'*Histomonas meleagridis,* respectivement le *Trichomonas vaginalis. Trichomonas vaginalis* est un protozoaire flagellé se développant chez l'humain ayant une localisation essentiellement uro-génitale. Il entraîne une maladie sexuellement transmissible et cosmopolite désignée trichomonose.

Chez la femme, les symptômes cliniques se présentent sous la forme d'une vulvo-vaginite aiguë avec une leucorrhée spumeuse jaune vert continuelle et nauséabonde, de même qu'un prurit vulvaire avec sensation de brûlure. Les complications sont la néoplasie cervicale et l'accouchement prématuré.

Chez l'homme, la trichomonose est asymptomatique, ce qui favorise la dissémination de la maladie. Il peut y avoir urétrite, épididymite, prostatite, voire stérilité.

Les produits aujourd'hui prescrits appartiennent à la famille des imidazolés et sont notamment le métronidazole, l'omidazole, le secnidazole, le ténonitrozole, et le tinidazole. Bien qu'efficaces, ces produits doivent être utilisés avec certaines précautions, en particulier chez la femme enceinte ou la femme qui allaite.

De manière générale, le problème à résoudre est donc de mettre au point une composition qui puisse être efficace pour la prophylaxie et le traitement des pathologies humaines ou animales impliquant des protozoaires flagellés et en particulier *Histomonas meleagridis* et *Trichomonas vaginalis,* qui soit en outre non cytotoxique et non mutagène.

De manière tout à fait surprenante, le Demandeur a constaté et démontré que le fenugrec présentait des propriétés parasiticides ou parasitostatiques vis-à-vis des protozoaires précédemment identifiés et plus généralement de tous les protozoaires flagellés appartenant au Phylum des Metamonada présentant des structures et physiologies similaires à celle de *Histomonas meleagridis* et *Trichomonas vaginalis.*

Le document JP 2008011731 décrit une composition contenant un extrait de fenugrec destinée à traiter la diarrhée du porcelet. Le document FR-A-2 833 813 décrit une composition contenant un extrait de fenugrec, destinée au traitement de la coccidiose.

L'invention a tout d'abord pour objet l'utilisation d'un extrait de fenugrec pour l'obtention d'une composition destinée au traitement préventif ou curatif des pathologies humaines ou animales impliquant des protozoaires flagellés appartenant au Phylum des Metamonada et en particulier, *Histomonas meleagridis* et *Trichomonas vaginalis.*

Dans un mode de réalisation avantageux, les protozoaires flagellés concernés par l'invention appartiennent au Phylum des Metamonada, incluant la classe des Parabasalia et des Eopharyngia.

En particulier, l'extrait est utilisé pour le traitement de l'histomonose des gallinacés, en particulier les volailles et notamment la dinde, et pour le traitement de la trichomonose chez l'homme et/ou la femme.

Le fenugrec, désigné sous l'appelation *Trigonella foenum-graecum* appartient à la famille des légumineuses. Originaire d'Afrique du Nord et du bassin méditerranéen, cette plante annuelle est cultivée depuis longtemps en Asie, notamment en Inde et en Chine. Le fenugrec trouve de nombreuses applications chez l'homme ou l'animal. Il peut en particulier être utilisé traditionnellement comme tonique pour stimuler l'appétit et améliorer la digestion, comme orexigénique ou encore pour soulager l'irritation des voies respiratoires. Plus récemment, les recherches et études cliniques ont montré que le fenugrec pouvait contribuer à la régulation du taux de glucose sanguin en cas de diabète.

Selon l'invention, la plante entière ou toutes les parties de la plante peuvent être utilisées.

Dans un premier mode de réalisation, l'extrait est obtenu à partir de la graine. Les graines sont avantageusement des graines détégumentées, qui sont micronisées (taille de l'ordre de 50 µm) ou atomisées (taille inférieure à 50 µm).

Dans un second mode de réalisation, l'extrait est obtenu à partir de germes préalablement séparés de la graine après germination.

En pratique, les graines détégumentées ou les germes sont mis à macérer sous agitation à température ambiante et la solution est ensuite centrifugée pour ne récupérer que le surnageant.

Bien entendu, l'extrait de fenugrec peut être utilisé tel quel ou plus avantageusement formulé au sein d'une composition, notamment pharmaceutique plus complexe en présence d'excipients. Il peut également être incorporé au sein d'un complément alimentaire solide ou d'une boisson, notamment aqueuse.

Les ingrédients pouvant être utilisés dans la formulation, en particulier sous forme de complément solide, sont à titre d'exemple le blé, le mais, le soja, l'huile de soja, l'huile de palme, ou encore des sels minéraux, acides aminés, vitamines et autres sources de carbone, et plus généralement tout composé pouvant entrer dans la composition d'aliments.

Lorsqu'il est utilisé au sein d'une composition ou d'un complément alimentaire ou d'une boisson, l'extrait représente entre 0.1 et 5 % en pourcentage en poids de la composition, de préférence entre 0.5 et 5 % en pourcentage en poids de la composition.

Lorsque l'extrait est utilisé pour le traitement des parasites flagellés cités dans la présente demande, chez l'animal et qu'il se présente sous la forme d'un complément alimentaire solide, il représente entre 1 et 3g/kg, avantageusement 2g/kg de complément.

Lorsque l'extrait est utilisé pour le traitement des parasites flagellés cités dans la présente demande, chez l'animal et qu'il se présente sous la forme d'une eau de boisson, il représente entre 1 et 3g/l, avantageusement 2g/l de boisson.

Lorsque l'extrait est utilisé pour le traitement des parasites flagellés cités dans la présente demande, chez l'homme et qu'il se présente sous la forme d'une composition solide, il représente entre 1 et 3g/kg, avantageusement 2g/kg de la composition.

Lorsque l'extrait est utilisé pour le traitement des parasites flagellés cités dans la présente demande, chez l'homme et qu'il se présente sous la forme d'une composition liquide, il représente entre 100 et 1000 µg/mL, avantageusement 500 µg/mL de la composition. Généralement, la composition est administrée oralement. Dans ce cas, la préparation est en général administrée en raison de 0.5 % de la composition.

Dans le cas de *Trichomonas vaginalis,* l'extrait est administré par voie vaginale.

Comme déjà dit, l'extrait de fenugrec peut être utilisé pour les traitements des pathologies dans lesquels les protozoaires flagellés sont impliqués et notamment en plus des protozoaires précités, les protozoaires faisant partie de la liste suivante :
- *Tetratrichomonas gallinarum, Trichomonas gallinae* qui sont des parasites intestinaux de gallinacés,
- *Trichomonas foetus,* parasite des voies génitales du bovin,
- *Trichomonas equi,* parasite intestinal du cheval,
- *Spironucleus vortens* parasite des poissons salmonidés,
- *Hexamita meleagridis* parasite chez les gallinacés,
- *Giardia intestinalis* parasite chez l'homme.

L'invention et les avantages qui en découlent ressortiront bien des exemples qui suivent à l'appui des figures annexées.
La figure 1 est une représentation graphique de l'activité *in vitro* de l'extrait de l'invention sur d'*H meleagridis.*
La figure 2 est une représentation graphique comparant l'activité de l'extrait de l'invention sur *H meleagridis* par rapport au produit de référence.
La figure 3 est une représentation graphique comparant l'efficacité de l'extrait de l'invention par rapport à celle du dimétridazole sur la croissance d'*H meleagridis.*
La figure 4 est une représentation graphique comparant les effets de l'extrait de l'invention avec ceux du DMZ sur la flore bactérienne associée à *H meleagridis.*
La figure 5 est une représentation graphique comparant l'efficacité de l'extrait de l'invention par rapport à celle du dimétridazole sur la croissance d' *H meleagridis* après reprise dans du milieu frais.
La figure 6 est une représentation graphique comparant les effets de l'extrait de l'invention avec ceux du DMZ sur la flore bactérienne associée à *H meleagridis* après 72h.
La figure 7 est une représentation graphique comparant la cytotoxicité de l'extrait de l'invention avec des molécules de référence à 24H (figure 7a) et 48H (figure 7b).
La figure 8 est une représentation graphique de l'efficacité de l'extrait de l'invention sur *Trichomonas vaginalis* à 24 (figure 8a)et 72h (figure 8a).

### 1/ Matériel et méthode

### 1.1. Préparation de l'extrait

L'extrait de fenugrec est obtenu par broyage à partir de graines détégumentées micronisées (grains de la taille égale à 50 µm) et est dénommé par la suite S520. 1 g de cette poudre est repris dans 5 mL d'eau stérile (concentration de 200 mg/mL).

La solution est laissée une nuit sous agitation à température ambiante puis centrifugée 10 min à 16100g à 4°C.

Le surnageant est récupéré et centrifugé à nouveau 10 min à 16100g à 4°C.

Le surnageant est ensuite stocké en aliquots de 1 mL à -80°C.

### 1.2. Culture d'Histomonas meleagridis

La souche d'H. *meleagridis* a été isolée à partir de caeca de dindes infestées expérimentalement par des oeufs du ver *H. gallinarum.* Les parasites sont cultivés à 39°C dans des tubes en verre scellés, contenant 3 mL de milieu M199 supplémenté. Ils sont repiqués deux fois par semaine. Pour cela, 80 µl de pré-culture sont ajoutés à 3 mL de milieu frais préalablement chauffé au bain marie à 39°C.

### Milieu M199 supplémenté :

- M199 42 mL
- Serum de cheval 5 mL
- Solution d'amidon de riz 4 mL
   pH= 7,4

### Solution d'amidon de riz : Source de carbone

- amidon de riz 0,6 g
- NaCl 0,325 g
- NaHCO₃ 0,05 g
- CaCl₂ 0,015 g
- H₂O qsp 50 mL

### 1.3. Culture de la flore bactérienne associée à H. meleagridis

Prélèvement d'un aliquot de culture d' *H. meleagridis.*

Réalisation de 4 dilutions sérielles (10⁻⁴ à 10⁻⁷) en milieu Tryptone Soja liquide.

Etalement de 20µL de chacune des dilutions sur milieu Tryptone Soja gélosé.

Incubation à 37°C : une nuit.

### 1.4. Culture de Trichomonas vaginalis

La souche de *T. vaginalis* est fournie par l'ATCC 30243 (souche de référence).

Les parasites ont été cultivés dans du milieu Hollander filtré à 35°C. Ils ont été repiqués tous les 3 jours. Pour cela 80 µl de la culture sont ajoutés à 3 mL de milieu frais préalablement chauffé au bain marie à 35°C.

### Milieu Hollander :

- Trypticase 20,00 g
- Yeast extract 10,00 g
- Maltose 5,00 g
- L-ascorbic acid 1,00 g
- KCl 1,00 g
- KHCO₃ 1,00 g
- KH₂PO₄ 1,00 g
- K₂HPO₄ 1,00g
- FeSO₄ 7H₂O 0,18 g
- H₂O qsp 1000 mL
   pH 6,2

### 1.5. Numération des parasites

Elle se fait par comptage en cellule de Malassez après coloration au Bleu de Trypan 0,4%. Les 100 champs de la cellule sont comptés.

### 1.6. Etude de l'activité anti-histomonique

Le S520 est testé à différentes concentrations. Une numération des parasites, en triplicate, est réalisée après 72h d'incubation. Un témoin négatif est réalisé en remplaçant l'extrait par le solvant seul (de l'eau stérile). Pour étudier l'effet dose-dépendant, chaque test est réalisé en triplicate, avec des concentrations différentes, pour un comptage des parasites à 24h, 48h et 72h. Un témoin négatif est réalisé en remplaçant l'extrait par le solvant seul.

### 1.7. Etude de l'activité anti-trichomonique

Le S520 a été testé à 4 concentrations : 1 mg/mL, 500 µg/mL, 100 µg/mL, 50 µg/mL. Des numérations des cultures de *T. vaginalis* sont réalisées après 24h, 48h et 72h d'incubation avec les échantillons. Les expériences ont été réalisées en triplicate.

### 1.8 Test de cytotoxicité

### 1.8.1 Culture des cellules MRC5

Les cellules MRC5 (lignée primaire de fibroblastes pulmonaires foetaux humains) sont cultivées en boîtes de 25 ou 75 cm² dans du milieu MEM (Minimum Essential Medium, *Life Technologies Gibco*) supplémenté, dans une étuve à 37°C et % de CO₂.

Un volume de milieu MEM de 500 mL est supplémenté avec du sérum de veau foetal (10% final), de la glutamine 2mM (5 mL), des antibiotiques (5 mL de Pénicilline-Streptomycine soit 0,1 mU/mL, 0,5 mL d'Ampicilline soit 10 µg/mL, 0,25 mL de Gentamycine soit 25 µg/mL) et des antifongiques (5 mL de Fungizone soit 2,5 µg/mL).

Lorsque les cellules arrivent à confluence, elles sont trypsinées et réparties dans des plaques 96 puits à 1 à 2.10⁵ cellules/puits. Elles sont ensuite incubées à 37°C, 5% de CO₂ pendant 24h avant de lancer le test.

Les plaques sont réalisées en duplicate pour un dosage des protéines à 24 et 48h.

### 1.8.2. Dosage des protéines

Ce test consiste à cultiver des cellules en présence des extraits, puis à précipiter les protéines totales pour pouvoir les doser.

Les cellules MRC5 sont mises en présence de 200 µL de milieu MEM supplémenté contenant différentes concentrations des extraits à tester. Chaque extrait est testé à 3 concentrations : 2 mg/mL, 1 mglmL et 500 µg/mL. Chaque dilution est testée en triplicate afin de pouvoir réaliser une moyenne.

Deux témoins sont réalisés :
- un témoin négatif: cellules en présence de milieu MEM supplémenté uniquement
- un témoin positif: cellules en présence d'un mélange de milieu MEM supplémenté et de 50% de DMSO

Les plaques sont ensuite incubées à 37°C, 5% de CO₂ pendant 24 ou 48h. Après 24 ou 48h, le milieu contenant les extraits est éliminé et remplacé par du milieu frais. Les protéines totales sont précipitées par 50µL d'acide trichloroacétique 50% (TCA). Après 2h d'incubation à 4°C les puits sont lavés à l'eau du robinet. Une fois la plaque sèche, de la sulforhodamine 0,4% (SRB) est ajoutée dans chaque puits pour colorer les protéines. Après 20 min d'incubation, les puits sont rincés à l'acide acétique 1% et les protéines sont solubilisées dans du tampon Tris-Base 10mM, pH 10,5. Le contenu des puits est homogénéisé et la densité optique lue à 490 nm.

### 1.9 Test de mutation réverse

La mutagénicité des extraits est évaluée à l'aide d'un kit commercialisé par une société canadienne (EBPI) : Muta-Chromoplate^{™} Kit-S9. Ce kit de Chromotest est basé sur l'essai bactérien le plus généralement utilisé et validé de mutation réverse, connu sous le nom de test de Ames. L'essai utilise un mutant de *Salmonella typhimurium* portant une mutation de l'opéron contrôlant la biosynthèse de l'histidine. Quand ces bactéries sont exposées à des agents mutagènes, sous certaines conditions, une mutation réverse apparaît et les bactéries initialement auxotrophes pour l'histidine deviennent prototrophes. Les produits sont testés avec ou sans activation par du S9. Il s'agit d'un homogénat de foie de rat qui mime le métabolisme hépatique, et permet de tester le produit ainsi que ses métabolites.

La veille de l'essai, le lyophilisat de bactéries est réhydraté avec du milieu de culture puis incubé à 37°C pendant 16 à 18h. Le milieu réactionnel et le mélange S9 sont préparés selon les instructions fournies dans le kit. Le milieu réactif est ajouté dans tous les tubes contenant un extrait à tester. Le S9 n'est rajouté que dans les tubes où l'extrait est testé avec activation. 5 µL de *Salmonella typhimurium* (TA100) sont rajoutés dans chaque tube test à l'exception du contrôle de stérilité. 200 µL du mélange sont répartis dans chaque puits d'une plaque 96 puits : 1 plaque pour chaque contrôle et 2 plaques pour chaque extrait (une avec activation et une sans activation). Les plaques sont fermées par des couvercles, placées dans des sacs plastiques stériles pour maintenir l'hygrométrie et incubées 5 jours à 37°C.

Cinq témoins sont réalisés :
- Un contrôle de stérilité (Blank): uniquement le milieu réactionnel
- Un contrôle de réversion spontanée sans activation (Background 1): les bactéries et le milieu réactionnel
- Un contrôle de réversion spontanée avec activation (Background 2): les bactéries, le mélange S9 et le milieu réactionnel
- Un contrôle avec un mutagène sans activation (Standard Mutagen 1): les bactéries, une substance mutagène (azide de sodium NaN₃) et le milieu réactionnel
- Un contrôle avec un mutagène et avec activation (Standard Mutagen 2): les bactéries, une substance mutagène (2-amino-anthracene), le mélange S9 et le milieu réactionnel

### 2/ Résultats

### 2.1. Activité anti-histomonique du S 520

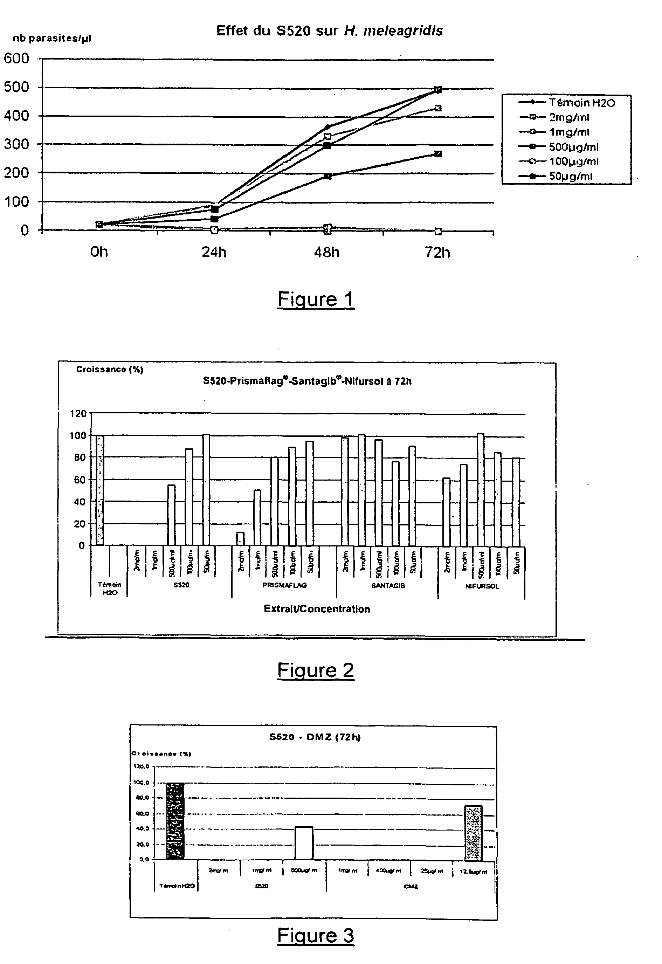

Comme le montre la figure 1, le S520 est histomonicide à partir de 1 mg/mL et dès 24h. Il ralentit la prolifération du parasite à 500 µg/mL (à 72h : 51% d'inhibition). L'effet est dose-dépendant

L'efficacité a été également comparée à celle de substances utilisées pour la lutte contre l'histomonose : le PrismaFlag^{®} (Santamix), le Santagib^{®} (Prisma) et le Nifursol (Figure 3). Les résultats sont exprimés en pourcentage de croissance par rapport au témoin

Comme il ressort de la figure 2, le PrismaFlag^{®} est histomonostatique à partir de 1 mg/mL (à 72h : 88% d'inhibition à 2 mg/mL et 50% d'inhibition à 1 mg/mL). Aucun effet significatif du Santagib^{®} n'a pu être mis en évidence sur la prolifération d'*H*. *meleagridis.* Le Nifursol est légèrement histomonostatique à 2 mg/mL (à 72h : 38% d'inhibition).

A concentrations égales, le S520 est donc l'extrait le plus efficace pour inhiber la prolifération d'*H*. *meleagridis.*

Le S520 a enfin été testé en parallèle du Dimétridazole (DMZ) Les résultats sont représentés sur la figure 3. Comme le montre cette figure, le DMZ est une substance parasiticide à 25 µg/mL, alors que le DMZ est parasitostatique.

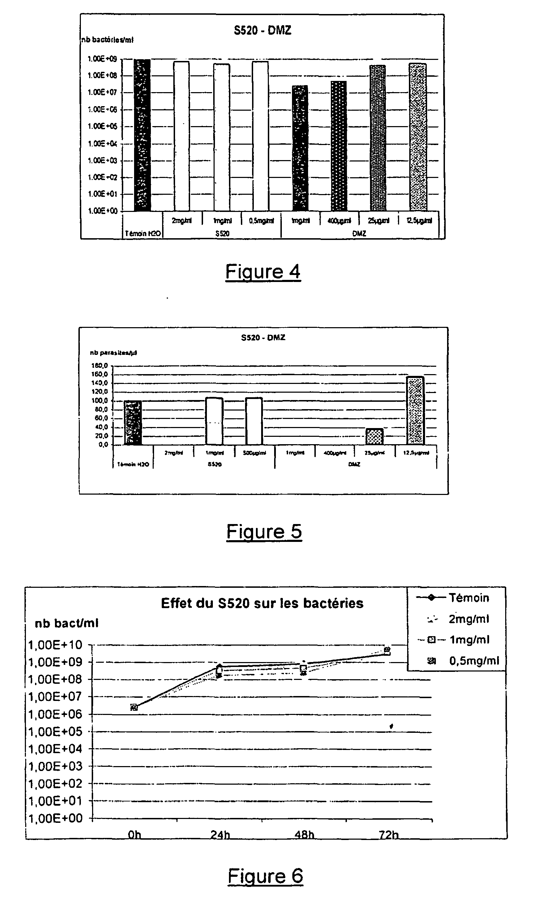

Les effets de différentes concentrations de S520 et de DMZ sur la flore bactérienne associée après 48h de culture sont ensuite comparés. Les résultats sont exprimés en nombre de bactéries par mL (figure 4). L'extrait S520 est histomonicide dès 1 mg/mL. Il est sans effet sur la flore bactérienne associée. Le Dimétridazole est histomonicide dès 25 µg/mL à 72h. En revanche il est bactéricide à partir de 400 µg/mL.

Après 72h de traitement avec le S520 et le DMZ, les parasites ont été remis dans du milieu frais pendant 96h pour analyser l'effet histomonocide (Fig. 5).

S520 : Pas de reprise de la culture à 2 mg/mL mais reprise à 1 mg/mL et 500 µg/mL. Ceci confirme que le S520 à 2 mg/mL est histomonicide. A 1 mg/mL quelques parasites survivent, il serait donc histomonostatique et non histomonicide.

DMZ : Pas de reprise de la culture à 1 mg/mL et 400 µg/mL mais reprise à 25 µg/mL mais (seulement environ 30% de croissance par rapport au témoin) et à 12,5 µg/mL.

Ceci confirme que le DMZ est histomonicide à partir de 400 µg/mL. Il ne serait qu'histomonostatique à 25µg/mL puisque la reprise de la culture indique que des parasites ont survécu au traitement. Il est à noter qu'à la concentration de 400 µg/mL, le DMZ a une action sur la flore associée (Fig. 4).

L'étude effectuée, durant 72h, sur la flore bactérienne associée montre que les différentes concentrations du S 520 n'ont aucun effet sur cette flore (Fig. 6).

### 2.2. Test de cytotoxicité

Test effectué sur des cultures de cellules MRC5.

L'extrait a été comparé à trois substances de référence : PrismaFlag^{®}, Santagib^{®} et Nifursol.

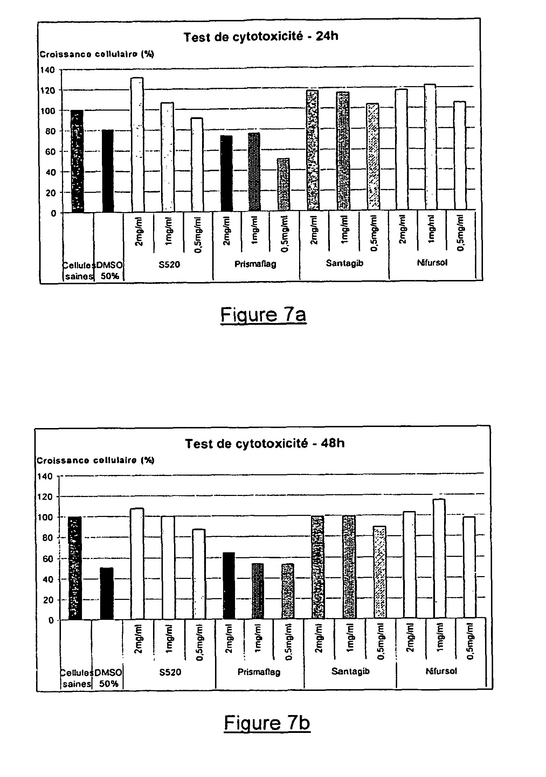

Nous avons testé 4 concentrations de chacun des extraits : 2 mg/mL, 1 mg/mL, 500 µg/mL et 100 µg/mL (Figures 7a et 7b).

Seul le PrismaFlag^{®} est considéré comme cytotoxique puisqu'il entraîne une croissance cellulaire inférieure à 60% par rapport au témoin à 500 µg/mL et ce dès 24h d'incubation avec les cellules. S 520 est non toxique pour les cellules humaines en culture. Il en est de même pour Santagib^{®} et Nifursol.

### 2.3. Test de mutation réverse

Pour que le S520 puisse être utilisé sur des élevages de volailles destinées à la production de viande il est indispensable de s'assurer qu'il n'est pas mutagène (et donc cancérigène). Le S520 à 2 mg/mL, 1 mg/mL et 500 µg/mL a donc été testé.

Les résultats sont présentés dans le tableau suivant :

| | | Background | S520 | | |
|---|---|---|---|---|---|
| | | | 2 mg/mL | 1 mg/mL | 500 µg/mL |
| % de mutations réverses | Sans activation | 54,2 | 14,6 | 7,3 | 10,4 |
| | Avec activation | 84,4 | 67,7 | 67,7 | 60,4 |

Sans activation par le S9, le taux de mutation réverse spontanée est de 54,2%. Pour les trois concentrations de S520 testées, un taux de mutation réverse spontanée inférieur est obtenu.

Avec activation par le S9, le taux de mutation réverse spontanée est de 84,4%. Cette fois encore le S520 donne un taux inférieur.

Le S520, activé ou non, n'est donc pas mutagène.

### 2.4 Activité anti-Trichomonas vaginalis du S520

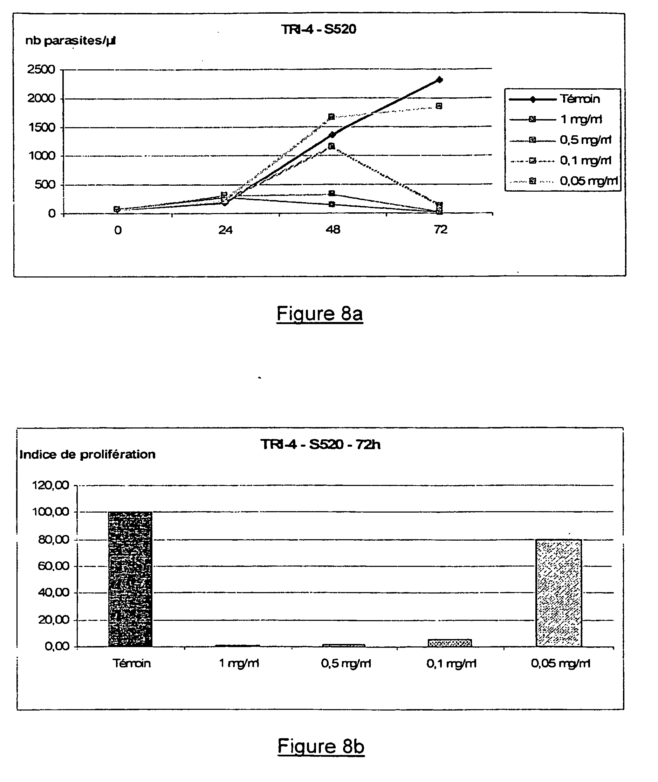

Un mix de milieu Hollander additionné de parasites a été préparé pour chaque test :
24 volumes de milieu Hollander supplémenté à 35°C + 1 volume de culture de *T. vaginalis.*
Le S520 a été testé à 4 concentrations différentes : 1 mg/mL, 500 µg/mL, 100 µg/mL, 50 µg/mL.
Des numérations des cultures de *T. vaginalis* ont été réalisées après 24h, 48h et 72h.
L'expérience a été menée en triplicate.
Les résultats (Fig. 8 et 9) montrent que le S520 est trichomonicide à partir de 500 µg/mL (1% de croissance par rapport au témoin à 72h).
Il est trichomonostatique à partir de 100 µg/mL (5% de croissance par rapport au témoin à 72h).

L'invention et les avantages qui en découlent ressortiront bien de la description qui précède, on note en particulier l'efficacité de l'extrait de fenugrec en particulier lorsqu'il se présente sous forme d'un extrait de graine micronisée détégumentée sur les parasites flagellés.

L'extrait peut donc être utilisé pour le traitement des pathologies telles que, en particulier, l'histomonose chez les gallinacés notamment la dinde. Il peut également être utilisé pour le traitement humain de la trichomonose. Dans tous les cas, il s'avère être non seulement efficace, mais également non mutagène et non cytotoxique.

## Revendications

1. Utilisation d'un extrait de fenugrec pour l'obtention d'une composition destinée au traitement préventif ou curatif des pathologies humaines ou animales impliquant des protozoaires flagellés appartenant au Phylum des Metamonada.

2. Utilisation selon la revendication 1, **caractérisée en ce que** les pathologies impliquent *Histomonas meleagridis* et *Trichomonas vaginalis.*

3. Utilisation selon la revendication 1 pour le traitement de l'histomonose des gallinacés, en particulier la dinde.

4. Utilisation selon la revendication 1 pour le traitement de la trichomonose chez l'homme et/ou la femme.

5. Utilisation selon la revendication 1, **caractérisée en ce que** l'extrait est obtenu à partir de graines détégumentées, micronisées ou atomisées.

6. Utilisation selon la revendication 1, **caractérisée en ce que** l'extrait est obtenu à partir de germes préalablement séparés de la graine après germination.

7. Utilisation selon la revendication 1, **caractérisée en ce que** l'extrait est un extrait aqueux.

8. Utilisation selon la revendication 1, **caractérisée en ce que** l'extrait est incorporé au sein d'un complément alimentaire ou d'une boisson et représente entre 0.1 et 5 % en pourcentage en poids de la composition.

9. Utilisation selon la revendication 1, **caractérisée en ce que** le parasite est choisi dans le groupe comprenant *Tetratrichomonas gallinarum, Trichomonas gallinae, Trichomonas foetus, Trichomonas equi, Spironucleus vortens, Hexamita meleagridis, Giardia intestinalis.*

## Patentansprüche

1. Verwendung eines Bockshornkleeextrakts zur Gewinnung einer Zusammensetzung, die zur präventiven oder kurativen Behandlung menschlicher oder tierischer Erkrankungen bestimmt ist, an denen die Protozoen Geißeltierchen beteiligt sind, die zum Stamm der Metamonada gehören.

2. Verwendung nach Anspruch 1, **dadurch gekennzeichnet, dass** an den Erkrankungen *Histomonas meleagridis* und *Trichomonas vaginalis* beteiligt sind.

3. Verwendung nach Anspruch 1 zur Behandlung der Histomonose von Hühnervögeln, insbesondere des Truthahns.

4. Verwendung nach Anspruch 1 zur Behandlung der Trichomonose beim Mann und/oder der Frau.

5. Verwendung nach Anspruch 1, **dadurch gekennzeichnet, dass** der Extrakt aus geschälten, mikronisierten oder atomisierten Samen gewonnen wird.

6. Verwendung nach Anspruch 1, **dadurch gekennzeichnet, dass** der Extrakt aus zuvor nach der Keimung vom Samen getrennten Keimen gewonnen wird.

7. Verwendung nach Anspruch 1, **dadurch gekennzeichnet, dass** der Extrakt ein wässriger Extrakt ist.

8. Verwendung nach Anspruch 1, **dadurch gekennzeichnet, dass** der Extrakt in ein Nahrungsergänzungsmittel oder ein Getränk eingebracht ist und zwischen 0,1 und 5% des Gewichtsanteils der Zusammensetzung ausmacht.

9. Verwendung nach Anspruch 1, **dadurch gekennzeichnet, dass** der Parasit aus der Gruppe ausgewählt ist, die *Tetratrichomonas gallinarum, Trichomonas gallinae, Trichomonas foetus, Trichomonas equi, Spironucleus vortens, Hexamita meleagridis, Giardia intestinalis* umfasst.

## Claims

1. Use of an extract of fenugreek to obtain a composition for the preventive or curative treatment of human or animal diseases involving flagellated protozoa belonging to the Metamonada phylum.

2. Use according to claim 1, **characterised in that** the diseases involves *Histomonas meleagridis* and *Trichomonas vaginalis.*

3. Use according to claim 1 for the treatment of histomoniasis of galliform birds, in particular turkeys.

4. Use according to claim 1 for the treatment of the trichomoniasis in men and/or women.

5. Use according to claim 1, **characterised in that** the extract is obtained from dehusked, micronised or atomised seeds.

6. Use according to claim 1, **characterised in that** the extract is obtained from germs previously separated from seeds after germination.

7. Use according to claim 1 **characterised in that** the extract is an aqueous extract.

8. Use according to claim 1, **characterised in that** the extract is incorporated into a dietary supplement or a drink and makes up between 0.1 and 5% by weight of the formulation.

9. Use according to the claim 1, **characterised in that** the parasite is chosen from the group including *Tetratrichomonas gallinarum, Trichomonas gallinae, Trichomonas foetus, Trichomonas equi, Spironucleus vortens, Hexamita meleagridis, Giardia intestinalis.*
